# EUROPÄISCHE PATENTSCHRIFT

(11) **EP 0 586 789 B1**
(45) Veröffentlichungstag und Bekanntmachung des Hinweises auf die Patenterteilung: **24.11.1999**
(21) Anmeldenummer: 93105630.3
(22) Anmeldetag: 06.04.1993
(51) Int. Cl.: G01N 1/28, B01D 61/00, B01L 3/00

(54) **Gleichmässig verteilte Anreicherung einer in Lösung vorliegenden Substanz auf der feinporigen Seite einer asymmetrisch porösen Membran**
Uniformly distributed enrichment of a dissolved substance at the fine pored side of an asymmetric porous membrane
Enrichissement réparti uniformément d'une substance en solution sur la face à pores minces d'une membrane poreuse asymétrique

(30) Priorität: 11.04.1992 DE 4212280
(43) Veröffentlichungstag der Anmeldung: 16.03.1994
(73) Patentinhaber: Roche Diagnostics GmbH, 68298 Mannheim (DE)
(72) Erfinder: Harttig, Herbert, Dr., W-6701 Altrip (DE); Merdes, Hartmut, Dr., W-6900 Heidelberg 1 (DE); Lange, Hans, W-6840 Lampertheim (DE); Zeiler, Manfred, W-6822 Altlussheim (DE)

(56) Entgegenhaltungen:
- EP-A- 0 407 800
- PATENT ABSTRACTS OF JAPAN vol. 15, no. 31 (C-0798) 2. November 1990 & JP-A-02 268 819 (UBE IND LTD)

## Beschreibung

Die Erfindung betrifft den Einsatz asymmetrisch poröser Membranen in Analysenverfahren und Testträgern zur Durchführung solcher Verfahren.

Als Testträger werden solche festen Analysemittel, vor allem in Form flacher Streifen oder Plättchen bezeichnet, die für Untersuchungen, insbesondere von biologischen Flüssigkeiten, wie Blut, Plasma, Serum, Urin etc., benötigte Reagenzien tragen. Die Trägermaterialien selbst sind fest und enthalten in der Regel auch die Reagenzien in fester Form. Als Trägermaterialien sind beispielsweise faserige Gebilde, wie Papier, Vliese, Gewebe, Gewirke, Netze etc., in der zu untersuchenden Flüssigkeit quellbare oder lösliche Filme oder poröse Membranen bekannt. Die für die Durchführung der Bestimmung von Analyten in Flüssigkeiten, wie z. B. Körperflüssigkeiten erforderlichen Reagenzien, können auf die Trägermaterialien durch Imprägnierung entsprechender Lösungen oder Beschichtung mittels entsprechender reagenzienhaltiger streichfähiger Massen und anschließende Trocknung hergestellt werden. Alternativ können natürlich auch die Trägermaterialien selbst bei ihrer Herstellung mit den erforderlichen Reagenzien versetzt werden. Beispielsweise können Filme oder Membranen aus gießfähigen Lösungen oder Suspensionen hergestellt werden, die bereits die für die Bestimmung eines Analyten notwendigen Reagenzien enthalten.

Bei Aufgabe einer zu untersuchenden Flüssigkeit auf einen solchen Testträger findet auf bzw. in dem Testträger eine Reaktion zwischen dem in der Probenflüssigkeit zu bestimmenden Analyt und den in dem Trägermaterial befindlichen Reagenzien statt. Die Reaktionsprodukte werden bestimmt und bilden ein Maß für die Menge des Analyten in der zu untersuchenden Probenflüssigkeit.

US-Patent 3,607,093 beschreibt beispielsweise einen Testträger zur Untersuchung biologischer Flüssigkeiten, der eine flüssigkeitsdurchlässige Membran enthält, die zumindest in Teilen ein diagnostisches Reagenz in fester Form, d. h. als trockene Substanz enthält. Die Membran selbst sollte so beschaffen sein, daß zumindest ein Oberflächenteil für größere Partikel, wie beispielsweise Erythrozyten, undurchlässig ist. Wie dem experimentellen Teil zu entnehmen ist, wird die Membran mit einer Lösung des für die Untersuchung biologischer Flüssigkeiten benötigten Reagenzes imprägniert und getrocknet. Bei Aufgabe der zu untersuchenden Flüssigkeit und gegebenenfalls nach Abwischen überschüssiger Flüssigkeit, wird bei Anwesenheit des zu bestimmenden Analyten in der Probenflüssigkeit eine Farbänderung der Membran beobachtet.

EP-A-0 345 781 betrifft einen Testträger zur Untersuchung von Flüssigkeiten. Er enthält eine asymmetrisch-poröse Membran, die ein oder mehrere Reagenzien trägt, die in Gegenwart des zu bestimmenden Analyten eine nachweisbare Substanz erzeugen. Zur Bestimmung eines Analyten in einer Flüssigkeit wird die Probenflüssigkeit auf die großporige Oberfläche der Membran aufgegeben, während die Vermessung von der feinporigen Oberfläche her erfolgt. Als besonders vorteilhaft wird die "BTS Asymmetric Membrane" von Filtrite (San Diego, Kalifornien, USA) beschrieben, da hier zelluläre Blutbestandteile abgetrennt werden und die Reagenz/ Analyt-Reaktion überall in der Membran abläuft. Zur Auswahl der Reagenzien, die bei Reaktion mit dem zu bestimmenden Analyt eine nachweisbare Substanz erzeugen, wird Wert auf für die Detektion wichtige Eigenschaften gelegt, beispielsweise Farbe, Chemilumineszenz etc., solange das Reagenz in der Membran ausreichend stabil ist.

EP-A-0 407 800 ist auf einen Teststreifen zur Analyse von Substanzen in biologischen Flüssigkeiten gerichtet. Er enthält eine asymmetrisch poröse Membran, die vorteilhafterweise aus einer Polymerlösung hergestellt wird, die 1 bis 4 Gew.-% eines anionischen Tensids enthält. Die Membran trägt die für die Analytbestimmung notwendigen Reagenzien. Zur Durchführung der Analyse wird die zu untersuchende Flüssigkeit auf die großporige Seite aufgegeben. Die Vermessung der Reaktionsprodukte erfolgt von der feinporigen Seite der Membran aus.

Keine der Schriften des Standes der Technik beschreibt eine Anreicherung der nachweisbaren Substanz, sei sie farbig, chemilumineszierend etc., auf einer der bezüglich ihrer Porengröße unterschiedlichen Oberflächen asymmetrisch poröser Membranen. Überraschenderweise wurde jetzt gefunden, daß eine Substanz auf der feinporigen Seite einer asymmetrisch porösen Membran gleichmäßig verteilt angereichert werden kann, wenn diese Substanz in Form einer Membran-benetzenden Lösung mit der Membran in Kontakt gebracht wird und wenn die Substanz nicht oder nicht wesentlich an der Membran adsorbiert wird.

Gegenstand der Erfindung ist deshalb die Verwendung einer asymmetrisch porösen Membran gemäß Anspruch 1.

Schließlich ist Gegenstand der Erfindung ein Verfahren zur Bestimmung einer Substanz gemäß Anspruch 3.

Ein besonders vorteilhafter Gegenstand der Erfindung ist ein wie vorstehend beschriebenes Verfahren, welches dadurch gekennzeichnet ist, daß die zu bestimmende Substanz Hämoglobin ist sowie ein hierfür geeigneter Testträger gemäß Anspruch 8.

Der Begriff "asymmetrisch porös" ist dem Fachmann allgemein bekannt und gebräuchlich (vgl. beispielsweise EP-A-0 407 800 oder EP-A-0 345 781). In der Regel versteht man hierunter einen durchgehend porösen Polymerfilm mit zwei sich gegenüberliegenden Oberflächen, wobei die Poren einer Oberfläche größer sind als die der gegenüberliegenden Oberfläche. Erfindungsgemäß sind solche asymmetrisch porösen Membranen bevorzugt, die einen Asymmetriefaktor von mehr als 10, besonders bevorzugt mehr als 100 aufweisen. Der Asymmetriefaktor gibt hierbei das Verhältnis der Porengröße auf der großporigen Oberfläche zu der Porengröße auf der feinporigen Oberfläche an. Erfindungsgemäß sind solche asymmetrisch porösen Membranen bevorzugt einsetzbar, bei denen die Porengröße auf der feinporigen Seite 0,003 - 3 µm beträgt.

Asymmetrisch poröse Membranen sind aus dem Stand der Technik bekannt, beispielsweise aus US-Patent 4,774,039; US-Patent 4,629,563 oder auch aus EP-A-0 345 781. Gemäß diesem Stand der Technik können asymmetrisch poröse Membranen vom Fachmann selbst hergestellt werden. Asymmetrisch poröse Membranen sind auch käuflich erhältlich, beispielsweise hat sich die BTS 25-Membran der Firma Memtec Timonium, Maryland, USA, erfindungsgemäß als vorteilhaft erwiesen. Es handelt sich hierbei um eine poröse Polysulfonmembran. Für den Gegenstand der Erfindung ebenfalls brauchbar haben sich mit Polyvinylpyrrolidon legierte Polyethersulfonmembranen, wie sie beispielsweise in EP-A-0 336 483 beschrieben sind, erwiesen. Solche Membranen werden beispielsweise unter der Bezeichnung PS 11 bzw. PS 21 von der Firma X-Flow B.V. (Enschede, Niederlande) vertrieben.

Um erfindungsgemäß einsetzbar zu sein, muß die asymmetrisch poröse Membran von der Flüssigkeit, die die Substanz, die auf der feinporigen Seite angereichert werden soll, enthält, benetzbar sein. Im Falle von wässrigen Flüssigkeiten, wie es Körperflüssigkeiten, wie Blut, Plasma, Serum, Urin, etc. sind, muß die Membran deshalb ausreichend hydrophil sein. Wenn das Membranmaterial selbst nicht ausreichend hydrophil ist, können Polymermembranen auch hydrophiliert werden. Hierzu können Membranen beispielsweise mit solchen Substanzen behandelt werden, die in Wasser quellbar, aber unlöslich sind. Aus US-Patent 4,413,074 ist beispielsweise bekannt, daß Hydroxyalkylcellulose zur Hydrophilierung von Polymermembranen eingesetzt werden kann. Polyvinylpyrrolidon ist ebenfalls ein mögliches Hydrophilierungsmittel.

Grundsätzlich gilt, daß eine Membran als durch die Flüssigkeit ausreichend benetzbar anzusehen ist, wenn ein Tropfen der Probenflüssigkeit mit einem Volumen von 20 µl bei Raumtemperatur innerhalb von weniger als 20 sec. vollständig in ein Membranstück von 15 mal 15 mm oder größer aufgesaugt wird und in der Membran gehalten wird bzw. wenn eine Aufgabe eines Flüssigkeitstropfens auf die grobporige Seite einer asymmetrisch porösen Membran zu einer Durchfeuchtung der Memban im Aufgabebereich in ihrer gesamten Dicke führt.

Erfindungsgemäß einsetzbar sind alle benetzbaren Membranen, an die die Substanz, die auf der feinporigen Seite angereichert werden soll, nicht adsorbiert. Ob ein bestimmtes Membran-Substanzpaar diese Bedingung erfüllt, kann einfach geprüft werden. Hierzu wird die zu prüfende Substanz in dem Lösungsmittel gelöst, mit dem auch der Anreicherungseffekt erzielt werden soll, beispielswiese im Fall wässriger Lösungen in Wasser. Die Lösung wird durch eine oder mehrere Lagen der in Frage kommenden Membran geschickt und anschließend überprüft, ob die Konzentration der Substanzen in der Lösung vor und nach dem Durchtritt durch die Membran unterschiedlich ist. Für farbige Substanzen kann dies auf folgende Weise geschehen: Eine asymmetrisch poröse Membran wird in 5 Lagen so in einen Membranfilterhalter (beispielsweise der Firma Sartorius, Göttingen, Deutschland) ohne Glasfilter gespannt, und so auf eine Saugflasche gesetzt, daß die großporige Seite der Membran jeweils nach oben zeigt. Die zu untersuchende Substanzlösung wird auf die großporige Seite der Membran im Filterhalter gegeben und unter Anlegen eines Unterdruckes durch die Membran gesaugt. Nach Ihrem Durchtritt durch die Membran wird die Flüssigkeit photometrisch auf ihre optische Dichte hin geprüft und der Wert mit dem der ursprünglichen Lösung verglichen. Adsorption der gelösten Substanz an die Membran hat dann stattgefunden, wenn sich die optische Dichte der Lösung vor und nach dem Durchtritt durch die Membran deutlich unterscheidet. Von einem deutlichen Unterschied kann dann geredet werden, wenn die optische Dichte der Lösung nach dem Durchtritt durch die Membran mindestens etwa 15 % geringer ist als die optische Dichte der ursprünglichen Lösung, wobei auf die Einhaltung folgender Randbedingungen geachtet werden sollte:

Konzentration der Substanz in der Lösung etwa 1-100 mg/l, Menge der durchzusaugenden Flüssigkeit 8 ml, Anzahl der Membranscheiben 5 Stück, Durchmesser der Membranscheiben im Membranfilterhalter etwa 60 mm, Dicke der einzelnen Membranscheiben etwa 110 bis 150 µm und Schnelligkeit des Durchsaugvorgangs etwa 0,25 ml/Sekunde.

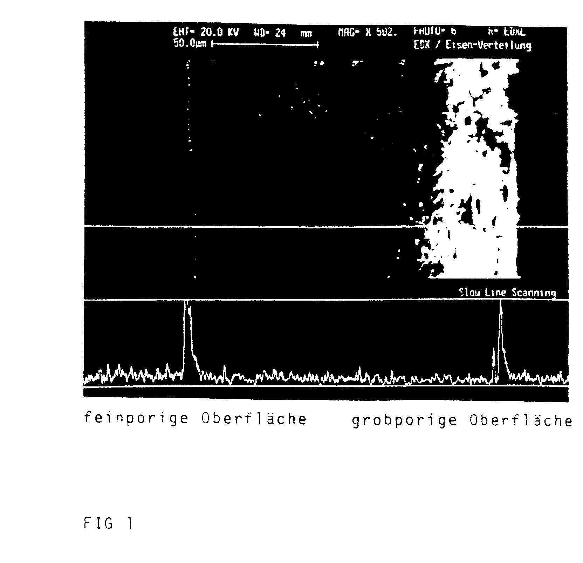

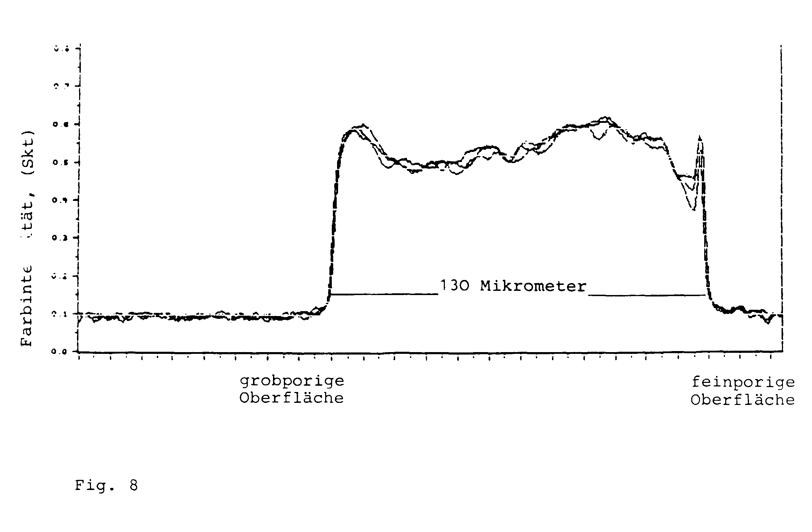

Substanzen, die nicht oder nicht wesentlich (weniger als 15 % nach vorstehend beschriebenem Prüfmodell) an der asymmetrisch porösen Membran adsorbiert werden, werden deutlich auf der feinporigen Seite der asymmetrisch porösen Membran angereichert. Ein entsprechendes Konzentrationsprofil ist Fig. 1, 2 und 3 zu entnehmen. Diese Figuren sind in Beispielen 2 und 3 näher erläutert. Ein Konzentrationsprofil, das das Verhalten einer Substanz auf einer asymetrisch porösen Membran zeigt, die an der Membran adsorbiert und nicht auf der feinporigen Seite der Membran angereichert wird, ist in Figur 8 dargestellt. Diese Figur ist in Beispiel 10 näher erläutert.

Um eine Substanz gleichmäßig verteilt auf der feinporigen Seite einer asymmetrisch porösen Membran anzureichern, wird erfindungsgemäß eine Membran-benetzende Lösung der Substanz mit der asymmetrisch porösen Membran in Kontakt gebracht. Dies kann durch Eintauchen der Membran in die Lösung oder zweckmäßigerweise durch Aufgeben der Lösung auf die Membran erfolgen. Bei Aufgabe der Lösung auf die Membran ist es zur Erreichung des Anreicherungseffektes gleichgültig, ob die Lösung auf die feinporige oder auf die grobporige Seite aufgegeben wird.

Das vorstehend beschriebene Verfahren zur gleichmäßig verteilten Anreicherung einer Substanz im wesentlichen auf einer Seite einer Membran kann für ein Verfahren zur trägergebundenen Bestimmung eines Analyten benutzt werden. Hierzu wird eine Lösung der zu bestimmenden Substanz mit einer asymmetrisch porösen Membran, die durch die Lösung benetzbar ist, kontaktiert und auf der Membran von der feinporigen Seite her bestimmt. Wie bereits zuvor ausgeführt, ist es erfindungsgemäß notwendig, daß die zu bestimmende Substanz nicht oder nicht wesentlich an der Membran adsorbiert wird. In der Regel wird die Lösung der zu bestimmenden Substanz auf die großporige Seite der Membran aufgegeben. Die zu bestimmende Substanz kann aber auch durch in oder auf der Membran befindliche Reagenzien oder Stoffe, die in einer oder mehreren Schichten über der Membran angeordnet sind, freigesetzt oder gebildet werden. Im Grunde genommen sind für solche trägergebundenen Bestimmungsverfahren Testträgeraufbauten einsetzbar, wie sie aus dem Stand der Technik bekannt sind, wobei jedoch erfindungsgemäß die Schicht, die vermessen wird, sei es visuell, optisch, reflexionsphotometrisch etc., eine asymmetrisch poröse Membran ist, wie sie vorstehend charakterisiert ist.

Erfindungsgemäß eignet sich das Bestimmungsverfahren besonders gut für farbige Analyten. Falls die zu bestimmende Substanz selbst nicht farbig ist, kann sie durch entsprechende Reaktionen, wie sie aus der klinischen Analytik bekannt sind, in farbige Substanzen überführt werden oder in chemischen Reaktionen Anlaß zur Bildung farbiger Reaktionsprodukte geben, die ein Maß für den zu bestimmenden Analyt darstellen.

Für optische Bestimmungen, seien sie visuell oder apparativ, insbesondere für reflexionsphotometrische Bestimmungen, ist es vorteilhaft, wenn die erfindungsgemäß eingesetzte asymmetrisch poröse Membran nur eine geringe Naßtransparenz aufweist, d. h. der Reflexionsgrad der erfindungsgemäß besonders vorteilhaft einsetzbaren Membran sollte nach Benetzung mit dem Lösungsmittel der zu untersuchenden Lösung größer als 20 %, noch besser jedoch größer als 50 % sein. Auch in diesem Zusammenhang hat sich für die Untersuchung wässriger Lösungen, wie es Körperflüssigkeiten, wie Blut, Plasma, Serum, Urin etc. sind, Polysulfonmembranen, wie beispielsweise die BTS-Membran von Memtec Timonium, Maryland, USA, vorteilhaft erwiesen.

Die Kombination der Filtereigenschaft asymmetrisch poröser Membranen mit geringer Naßtransparenz erweist sich als besonders vorteilhaft, wenn flüssige Proben untersucht werden sollen, die gefärbte partikuläre Komponenten enthalten. So bringt die erfindungsgemäße Verwendung entsprechender asymmetrisch poröser Membranen große Vorteile beim Einsatz in Verfahren zur trägergebundenen Bestimmung von Substanzen in Vollblut. Bei Aufgabe von Vollblut auf die großporige Seite einer ausreichend hydrophilen Membran mit einem Asymmetriefaktor größer als 10, vorzugsweise größer als 100, wobei die Poren auf der feinporigen Seite der asymmetrisch porösen Membran etwa 0,003 bis 3 µm groß sind, werden die roten Blutkörperchen (Erythrozyten) daran gehindert, zur feinporigen Seite der Membran zu gelangen. Sie werden von Plasma oder Serum getrennt, welches die gelösten Probeninhaltsstoffe und gegebenenfalls gelöste Reagenzien und entsprechende Reaktionsprodukte durch Kapillarkraft zur feinporigen Seite der Membran transportiert. Die zu bestimmende Substanz, die bereits ursprünglich in der Probe vorlag oder auf der Membran durch Reagenzkontakt in der Probenflüssigkeit freigesetzt oder gebildet wird, wird hierbei auf der feinporigen Seite gleichmäßig verteilt und angereichert, wenn sie vom Membranmaterial nicht oder nicht wesentlich adsorbiert wird.

Auf diese Art und Weise sind empfindlichere Bestimmungen, als es ohne den Anreicherungseffekt der Fall wäre, möglich. Dadurch, daß gleichzeitig mit der Anreicherung auch eine gleichmäßige Verteilung der zu bestimmenden Substanz auf der feinporigen Seite der asymmetrisch porösen Membran erfolgt, können Messungen mit sehr kleinen Variationskoeffizienten durchgeführt werden.

Das erfindungsgemäße Verfahren zur trägergebundenen Bestimmung einer Substanz hat sich als besonders vorteilhaft erwiesen für die Bestimmung von Hämoglobin oder Hämoglobinderivaten aus Vollblut. Aus den Werten für die Hämoglobinkonzentration im Blut kann man auch auf den Hämatokrit, das ist der Anteil der zellulären Bestandteile am Volumen des Blutes, rückschließen.

Hämoglobin und Hämoglobinabkömmlinge, wie beispielsweise Hämiglobinrhodanid, Hämiglobincyanid, Hämiglobin, Oxihämoglobin oder alkalisches Hämatin werden bei Einsatz ausreichend hydrophiler asymmetrisch poröser Membranen auf der feinporigen Seite sehr stark angereichert.

Ein erfindungsgemäßer Testträger zur Bestimmung von Hämoglobin oder Hämoglobinderivaten enthält als wesentlichen Bestandteil eine asymmetrisch poröse Membran, die mit Wasser benetzbar ist, die das zu bestimmende Hämoglobin oder Hämoglobinderivat nicht wesentlich adsorbiert und die eine hämolysierende Substanz trägt oder eine solche Substanz in einer über der Membran angeordneten Schicht enthält.

Hämolysierende Mittel sind dem Fachmann bekannt. Beispielsweise können für den erfindungsgemäßen Testträger Detergenzien, wie beispielsweise anionische, kationische oder nichtionogene Detergenzien eingesetzt werden. Beispiele für anionische Detergenzien sind Natriumnonylsulfat, Natriumdodecylsulfat, Natriumdodecylsulfonat, Natriumdeoxycholat, Natriumdioctylsulfosuccinat (DONS) oder Natriumdiamylsulfosuccinat (DANS). Als kationische Detergenzien sind beispielsweise Cetylpyridiniumchlorid, Cetyldimethylethylammoniumbromid oder Cetyltrimethylammoniumbromid einsetzbar. Unter den nichtionogenen Detergenzien sind insbesondere Polyoxyethylenäther bekannt. Erfindungsgemäß kann als hämolysierendes Mittel eine Substanz oder eine Mischung aus mehreren Substanzen eingesetzt werden.

Der erfindungsgemäße Testträger kann das Hämolysemittel direkt auf der asymmetrisch porösen Membran tragen oder es kann sich in einer weiteren Schicht, die über der großporigen Seite der asymmetrisch porösen Membran angeordnet ist, befinden. Soll für das Hämolysemittel keine weitere Schicht eingesetzt werden, bringt man zweckmäßigerweise das Hämolysemittel durch Imprägnierung auf die asymmetrisch poröse Membran. Es ist auch denkbar, das Hämolysemittel in einer streichfähigen Paste auf die großporige Seite der Membran aufzubringen und dort zu trocknen. Bei Einsatz einer separaten Schicht kann ein geeignetes Trägermaterial ebenfalls mit einer Lösung des Hämolysemittels imprägniert oder mit einer streichfähigen Masse des Hämolysemittels beschichtet werden. Die Art des Trägermaterials spielt hierbei keine Rolle, solange es keine Störung der Bestimmungsreaktion verursacht. Als mögliche Störung könnte man sich beispielsweise die Adsorption von Hämoglobin an das Trägermaterial oder störende Reaktionen des Trägermaterials mit Hämoglobin oder Hämoglobinderivaten vorstellen. Als geignet hat sich beispielsweise ein Glasfaservlies erwiesen.

Als Zusatz zum hämolysierenden Mittel haben sich Puffersubstanzen sowie Substanzen, welche zu einer Oxidation bzw. Komplexierung des Hämoglobins führen, als vorteilhaft erwiesen. Als Puffersubstanzen kommen hierbei solche in Frage, die einen pH-Wert im Bereich von 2 - 12, vorzugsweise 6 - 8 einstellen können. Als besonders vorteilhaft sind diesbezüglich Phosphatpuffer (pH 5 - 8), Citratpuffer (pH 2 -8), Citrat-Phosphat-Borat-Puffer (pH 2 - 12) zu nennen.

Zur Oxidation von Hämoglobin können alle Substanzen eingesetzt werden, die Eisen oxidieren, nicht aber die Struktur des Hämoglobins zerstören. Als besonders vorteilhaft haben sich höherwertige Metallsalze und -komplexverbindungen erwiesen, wobei Kaliumhexacyanoferrat (III) ganz besonders bevorzugt ist. Zur Komplexierung des hierbei entstandenen Hämiglobin können Substanzen aus der Gruppe Halogenide und Pseudohalogenide eingesetzt werden. Besonders vorteilhaft ist die Verwendung von Cyaniden, Fluoriden oder Rhodaniden. Das erfindungsgemäße Verfahren ist jedoch auch für den Nachweis von nichtkomplexiertem Hämiglobin geeignet.

Bei einer vorteilhaften Ausführungsform eines erfindungsgemäßen Testträgers zur Bestimmung von Hämoglobin ist die asymmetrisch poröse Membran auf einem steifen Material, wie beispielsweise einer Plastikfolie angeordnet, damit der Testträger besser und einfacher handhabbar ist und um die asymmetrisch poröse Membran bei der Durchführung des Tests nicht mit den Fingern berühren zu müssen. Die asymmetrisch poröse Membran ist dabei so auf diesem Träger befestigt, daß entweder die Aufgabe der zu untersuchenden Probe oder die Bestimmung auf der Oberfläche der asymmetrisch porösen Membran erfolgen kann, die dem Träger zugewandt ist. Bei der Aufgabe der Probe auf der dem Träger zugewandten Oberfläche der asymmetrisch porösen Membran bedeutet dies, daß das steife Trägermaterial für die Probe durchlässig sein muß. Im einfachsten Falle befindet sich ein Loch in dem Träger und unter dem Loch ist die asymmetrisch poröse Membran so befestigt, daß die Probe durch das Loch auf die Membran aufgegeben werden kann.

Ist die Oberfläche der asymmetrisch porösen Membran, von der die Vermessung her erfolgen soll, dem Träger zugewandt, darf der Träger diese Vermessung nicht stören. Hierfür ist es beispielsweise denkbar, daß der Träger bei optischen Meßverfahren lichtdurchlässig ist. Eine weitere Möglichkeit besteht auch hier darin, daß sich ein Loch in dem Träger befindet und die Membran über diesem Loch auf dem Trägermaterial so befestigt ist, daß die entsprechende Oberfläche der asymmetrisch porösen Membran vermessen werden kann.

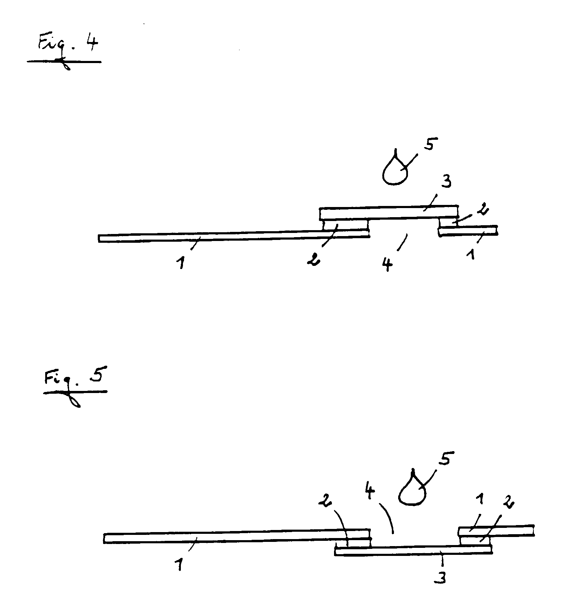

Vorteilhafte Ausführungsformen erfindungsgemäßer Testträger zur Bestimmung von Hämoglobin sind in Figuren 4, 5 und 6 dargestellt. Fig. 7 zeigt einen Testträger, mit dem die gleichmäßige Verteilung einer Substanz auf der feinporigen Seite einer asymmetrisch porösen Membran demonstriert werden kann.

Der Testträger gemäß Fig. 4 besteht aus einem Streifen (1) aus steifem Material, wie beispielsweise einer Plastikfolie mit einem Loch (4). Über dem Loch (4) befindet sich eine asymmetrisch poröse Membran (3), die mit einem beidseitig klebenden Band (2) auf dem Streifen (1) befestigt ist. Die asymmetrisch poröse Membran (3) ist so angebracht, daß ihre feinporige Seite zum Streifen (1) zeigt. Ähnliche Testträgeraufbauten sind beispielsweise aus EP-A-0 256 806 oder EP-A-0 407 800 bekannt. Im Gegensatz zum Stand der Technik enthält der erfindungsgemäße Testträger zur Bestimmung von Hämoglobin jedoch ein hämolysierendes Mittel sowie gegebenenfalls noch weitere Substanzen wie beispielsweise Puffersubstanz und/oder Hämoglobin oxidierende bzw. komplexierende Mittel in der asymmetrisch porösen Membran (3). Blut (5) wird auf die großporige Seite der asymmetrisch porösen Membran (3) gegeben, die Erythrozyten werden in der Membran (3) hämolysiert und das freigesetzte Hämoglobin oder entsprechende Hämoglobin-abkömmlinge, beispielsweise bei Anwesenheit von oxidierenden oder komplexierenden Substanzen als zusätzlichen Reagenzien, in der Membran (3) auf der feinporigen Seite der asymmetrisch porösen Membran (3) gleichmäßig verteilt und angereichert. Durch das Loch (4) wird die Konzentration des Hämoglobins bzw. der Hämoglobin-Abkömmlinge auf der feinporigen Seite der asymmetrisch porösen Membran (3) bestimmt.

Der Testträger gemäß Fig. 5 unterscheidet sich von demjenigen gemäß Fig. 4 dadurch, daß die asymmetrisch poröse Membran (3) so auf dem Streifen (1) befestigt ist, daß die großporige Oberfläche der Membran (3) dem Streifen (1) zugewandt ist.

Blut (5) wird durch das Loch (4) auf die großporige Seite der asymmetrisch poröse Membran (3) gegeben. Die Vermessung erfolgt von der dem Loch (4) gegenüberliegenden Seite von der feinporigen Oberfläche der Membran (3) aus.

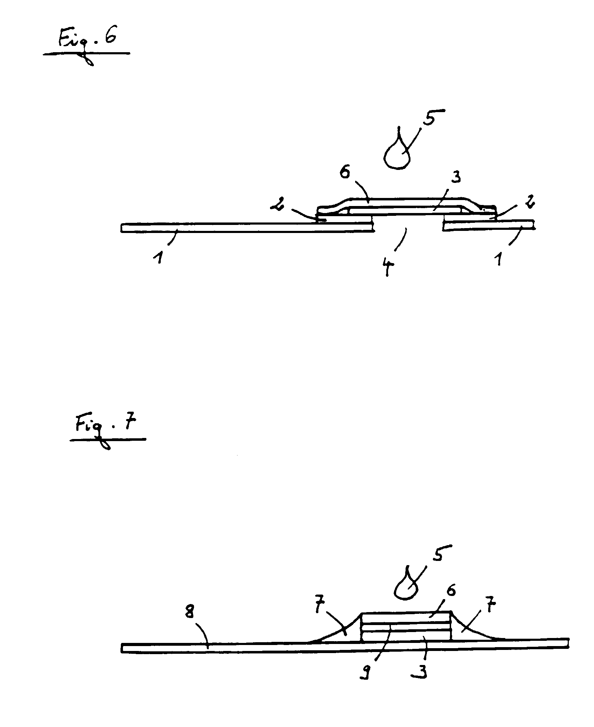

In Fig. 6 ist ein Testträger dargestellt, der über der großporigen Oberfläche der asymmetrisch porösen Membran (3) eine Schicht (6) trägt, die mit allen oder mit einem Teil der für den Test erforderlichen Reagenzien imprägniert sein kann. Beispielsweise kann diese Schicht (6) die für die Hämolyse der Erythrozyten erforderlichen Substanzen enthalten. Bei Aufgabe des Blutes (5) auf die Schicht (6) werden diese Substanzen gelöst und gelangen mit der Probenflüssigkeit in die Membran (3). Die Beobachtung der Nachweisreaktion erfolgt von der feinporigen Seite der Membran (3) durch das Loch (4).

In Fig. 7 ist ein Testträger dargestellt, bei dem sich auf einem Streifen (8) aus steifem, durchscheinendem Material mit Schmelzkleber (7) befestigt eine asymmetrisch poröse Membran (3) befindet, die so angebracht ist, daß die feinporige Oberfläche dem Streifen (8) zugewandt ist. Über der Membran (3) ist eine Schicht (6), beispielsweise ein Glasfaservlies so angebracht, daß sie die Membran (3) nicht berührt, durch Druck von oben jedoch mit ihr in Kontakt gebracht werden kann. In der Ausgangslage liegt deshalb ein Spalt (9) zwischen Membran(3) und Schicht (6) vor. Nach Aufgabe der flüssigen Probe auf Schicht (6) verweilt die Probe dadurch dort, ohne in die Membran (3) zu gelangen. Die Verweildauer ist frei wählbar. Erst nach Druck auf die Schicht (6), so daß diese Schicht mit der Membran (3) in Berührung gebracht wird, wird ein Flüssigkeitsübertritt von der Schicht (6) in die Membran (3) ermöglicht. Durch den durchscheinenden Streifen (8) hindurch kann die feinporige Seite der asymmetrisch porösen Membran (3) beobachtet werden. Die Konzentration einer nachweisbaren Substanz kann so durch den Streifen (8) hindurch vermessen werden.

Die Erfindung wird durch die folgenden Beispiele näher erläutert, die jedoch nicht als Beschränkung der Erfindung auf diese konkreten Ausführungsformen verstanden werden sollen.

In den Abbildungen wird folgendes gezeigt:
- Fig. 1:: Diagramm einer rasterelektronischen EDX-Mikroanalyse eines mit Kohlenstoff bedampften Querschnitts durch eine mit K₃[Fe(CN)₆] getränkte asymmetrisch poröse Membran.
- Fig. 2:: Darstellung der remissionsphotometrischen Farbintensität über den Querschnitt einer mit Tartrazin getränkten asymmetrisch porösen Membran
- Fig. 3:: Darstellung der remissionsphotometrischen Farbintensität über den Querschnitt einer mit Indigotin getränkten asymmetrisch porösen Membran.
- Fig. 4-6:: Querschnitte durch vorteilhafte Ausführungsformen erfindungsgemäßer Testträger.
- Fig. 7:: Querschnitt durch einen Testträger, mit dem die gleichmäßige Verteilung einer Substanz auf der feinporigen Seite einer asymmetrisch porösen Membran demonstriert wird.
- Fig. 8:: Darstellung der remissionsphotometrischen Farbintensität über den Querschnitt einer mit Bromthymolblau getränkten asymmetrisch porösen Membran.

### Beispiel 1

a) Auf die großporige Seite einer asymmetrisch porösen Membran (BTS 25, Hersteller Fa. Memtec Timonium, Maryland, USA) werden 10 µl verschiedener Farbstofflösungen gegeben.

### Herstellung der Farblösungen:

1. Indigotin (Hersteller Aldrich, Steinheim, Deutschland, Katalog-Nr. 22,929-6)
   Stammlösung: 20 mg/10 ml Wasser lösen
   Verdünnung: 10 µl Stammlösung in 20 ml Wasser lösen
   Konzentration der Farblösung = 1 mg/l
2. Toluidinblau (Hersteller Fluka, Buchs, Schweiz, Katalog-Nr. 89640)
   a) In Ethanol:
      Stammlösung: 20 mg/10 ml Ethanol lösen
      Verdünnung: 100 µl Stammlösung in 10 ml Ethanol lösen
      Konzentration der Farblösung = 20 mg/l
   b) In Wasser:
      Stammlösung: 20 mg/10 ml Wasser lösen
      Verdünnung: 100 µl Stammlösung in 10 ml Ethanol lösen
      Konzentration der Farblösung = 20 mg/l
3. Rhodamin B (Hersteller Aldrich, Steinheim, Deutschland, Katalog-Nr 25,242-5)
   Stammlösung: 12,5 mg in 10 ml Ethanol lösen
   Verdünnung: 100 µl Stammlösung in 10 ml Ethanol lösen
   Konzentration der Farblösung = 1,25 mg/l
4. Coomassie blue (Hersteller Serva, Heidelberg, Deutschland, Katalog-Nr. CJ42655)
   Stammlösung: 20 mg in 8 ml Ethanol lösen
   Verdünnung: 70 µl Stammlösung in 10 ml Ethanol lösen
   Konzentration der Farblösung = 17,5 mg/l
5. Tartrazin (Hersteller Serva, Heidelberg, Deutschland, Katalog-Nr. CJ19140)
   Stammlösung: 20 mg in 2 ml Wasser lösen
   Verdünnung: 50 µl Stammlösung in 10 ml Wasser lösen
   Konzentration der Farblösung = 50 mg/l
6. Safranin (Hersteller Aldrich, Steinheim, Deutschland, Katalog Nr. 10,214-8)
   a) In Ethanol:
      Stammlösung: 20 mg in 10 ml Ethanol lösen
      Verdünnung: 100 µl Stammlösung in 10 ml Ethanol lösen
      Konzentration der Farblösung = 20 mg/l
   b) In Wasser:
      Stammlösung: 20 mg in 10 ml Wasser lösen
      Verdünnung: 100 µl Stammlösung in 10 ml Wasser lösen
      Konzentration der Farblösung = 20 mg/l
7. Acid green 41 (Hersteller Aldrich, Steinheim, Deutschland, Katalog-Nr. 21,071-4)
   Stammlösung: 20 mg in 10 ml Ethanol lösen
   Verdünnung: 400 µl Stammlösung in 10 ml Ethanol lösen
   Konzentration der Farblösung = 80 mg/l
8. Bromthymolblau (Hersteller E. Merck, Darmstadt, Deutschland, Katalog-Nr. 3026)
   Stammlösung: 10 mg in 5 ml Puffer pH 9 lösen
   Verdünnung: 40 µl Stammlösung in 10 ml Puffer lösen
   Konzentration der Farblösung = 8 mg/l
9. Kaliumhexacyanoferrat III (Hersteller Aldrich, Steinheim, Deutschland, Katalog-Nr. 22,768-4)
   Stammlösung: 20 mg in 10 ml 0,1 N Phosphatpuffer, pH3 lösen
   Konzentration der Farblösung = 2000 mg/l
Aufgrund des Farbeindrucks, der nach Aufgabe der Lösung auf die großporige Seite auf der feinporigen Seite entsteht, kann beurteilt werden, ob eine Anreicherung auf der feinporigen Seite erfolgt (siehe Ergebnistabelle).

b) Zur Überprüfung auf Adsorption oder Nichtadsorption von Substanzen an Membranen werden Filtrationsexperimente durchgeführt. Dazu werden die Farblösungen aus a) durch mehrere Lagen Membranen gefiltert. Die Konzentration an Farbstoff in den Lösungen vor und nach der Filtration wird photometrisch bestimmt.

### Durchführung der Messungen

Von 10 ml der jeweiligen Farblösung wurden 2 ml abgenommen und in einer 10 mm Küvette mit einem UV/VIS Spektrometer, Modell 845 A, Fa. Hewlett Packard im Vergleich zum reinen Lösungsmittel vermessen.

Aus einer BTS25-Membran (Fa. Memtec Timonium, MD, USA) wurden Scheiben mit einem Durchmesser von 60 mm gestanzt. Pro Adsorptionsversuch wurde ein Stapel von 5 Membranscheiben, mit der grobporigen Seite nach oben, in einen Membranfilterhalter der Fa. Sartorius (Göttingen, BRD) gespannt. Die Glasfritte wurde vorher aus dem Filterhalter entfernt.

Der Filterhalter wurde auf eine Saugflasche gesetzt. Die verbliebenen 8 ml Farblösung wurden unter Anlegung eines geringen Unterdrucks durch die Membranlagen gesaugt. Das Filtrat wurde dann entsprechend der Ausgangslösung vermessen. Aus den Extinktionen wurden die Konzentrationen berechnet.

| Ergebnisse: | | | | | | | |
|---|---|---|---|---|---|---|---|
| Farbstoff | Lösemittel | Anreicherung | Extinktionen | | Konzentration | | % Unterschied |
| | | | Vor | Nach | Vor | Nach | |
| | | | Filtration in % | | Filtration in mg/l | | |
| Indigotin | Wasser | + | 0,055 | 0,050 | 1 | 0,9 | -8,78 |
| Toluidin blau | Ethanol | + | 1,124 | 1,08 | 20 | 19,22 | -3,9 |
| | Wasser | - | 0,47 | 0,392 | 20 | 16,68 | 1 -16,6 |
| Rhodamin B | Ethanol | + | 0,329 | 0,327 | 1,25 | 1,2 | -0,8 |
| Coomassie blue | Ethanol | + | 0,68 | 0,682 | 17,5 | 17,55 | +0,2 |
| Tartrazin | Wasser | + | 0,242 | 0,241 | 50 | 49,79 | -0,4 |
| Safranin | Ethanol | + | 0,79 | 0,79 | 20 | 20 | 0,0 |
| | Wasser | - | 1,59 | 1,33 | 20 | 16,73 | -16,35 |
| Acid green | Ethanol | + | 0,824 | 0,821 | 80 | 79,71 | -0,36 |
| Bromthymolblau | Puffer pH 9 | - | 0,289 | 0,163 | 8 | 4,51 | -43,6 |
| Kaliumhexacyanoferrat (III) | 0,1 N phosphatpuffer pH 3 | + | 0,383 | 0,382 | 2000 | 1995,3 | -0,23 |

Es zeigte sich, daß alle Farbstoffe, deren Konzentration weniger als 15 % abnahm, den gefundenen Anreicherungseffekt ergaben, während solche Farbstoffe, deren Konzentrationen stärker abnahmen, den Effekt nicht zeigten.

c) Prüfung, ob die getesteten Lösungen die Membran benetzen, erfolgt folgendermaßen: Ein Tropfen der Lösung mit 20 µl Volumen wird auf die grobporige Seite der 15 x 15 mm großen Membran aufgesetzt. Die Zeit, bis zum vollständigen Einsaugen wird gemessen. Der Versuch wiederholt, mit dem Unterschied, daß auf die feinporige Seite aufgegeben wird. Liegen beiden Zeiten unter 20 sec. gelten die Lösungen als benetzend.

### Beispiel 2

Eine asymmetrisch poröse Membran BTS 25 (Fa. Memtec Timonium, MD, USA) wurde mit einer Lösung von 1 % Natriumdodecylsulfat, 0,1N Phosphatpuffer pH 7 und 0,7 mmol/l K₃Fe(CN)₆ getränkt. Die Flüssigkeitsaufnahme betrug umgerechnet 115 ml/m². Die flüssigkeitsbeladene Membran wurde 30 Minuten bei 50° C getrocknet. Nach Kühlung mit flüssigem Stickstoff wurde die getränkte Membran gebrochen. Der Querschnitt wurde mit Kohlenstoff bedampft und in einem Rasterelektronenmikroskop mit EDX-Mikroanalyse der Fa. Cambridge, Nußloch, Deutschland auf Eisen untersucht.

Es kann gezeigt werden, daß sich Eisen auf der feinporigen Seite der Membran stark angereichert hat (Fig. 1). Dies wurde visuell bestätigt durch deutliche Unterschiede in der Gelbfärbung der feinporigen und der grobporigen Seite. Eisensignale auf der grobporigen Seite ergaben sich durch Blick auf die Oberfläche infolge einer leichten Schräglage der Probe in der Meßhalterung.

### Beispiel 3

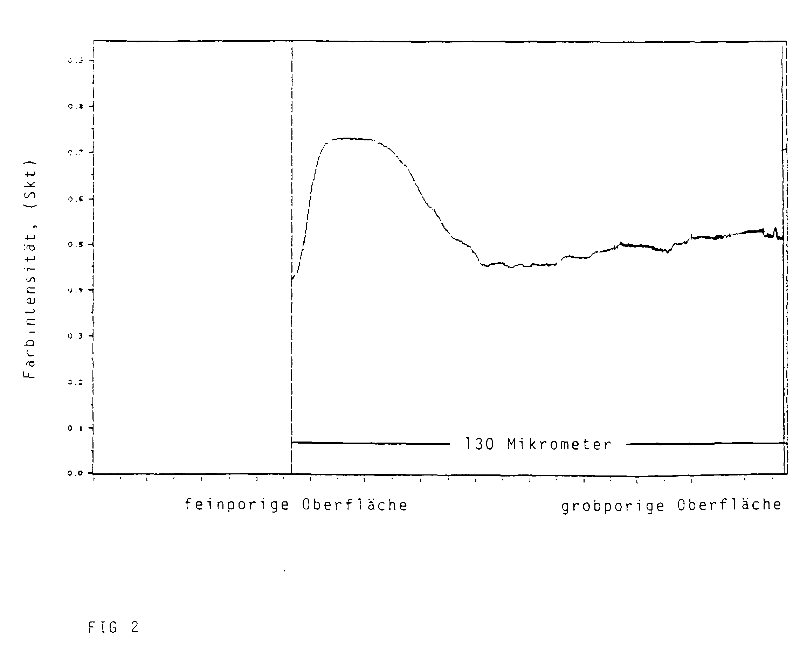

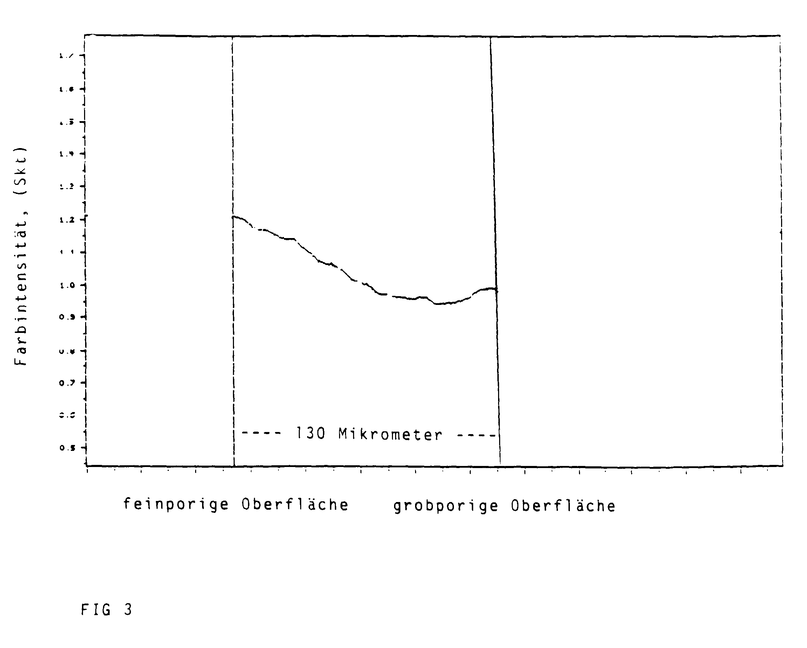

Zur Untersuchung von auf der feinporigen Seite einer asymmetrisch porösen Membran anreichernden Farbstoffen wurden Querschnitte von BTS 25-Membranen (Fa. Memtec Timonium, MD, USA) nach Kontakten mit den entsprechenden Farblösungen aus Beispiel 1 hergestellt. Die Membranen wurden dazu mit einer neuen Rasierklinge zerschnitten. Der Querschnitt wurde in einem Lichtmikroskop mit angeschlossenem Farbvideoprinter photografiert und als vergrößerter Videoprint ausgedruckt. Der Farbausdruck wurde anschließend auf einem remissionsphotometrischen Scanner, Elscript 400, Fa. Hirschmann, Deutschland abgescannt. Aus 4 Meßfahrten wurde ein Mittelwert gebildet. Damit konnte der optische Eindruck quantitativ dargestellt werden. Beispielhaft sind in Fig. 2 die Ergebnisse von Tartrazin und in Fig. 3 die Ergebnisse mit Indigotin als sich anreichernden Substanzen dargestellt. Die grobe Struktur auf der großporigen Seite täuscht im Diagramm mehr Farbe vor, als tatsächlich vorhanden ist, da Schwarz vom Scanner auch als Farbe interpretiert wird.

### Beispiel 4

10 µl Hämiglobincyanidlösung (7 g/dl) werden auf die grobporige Seite einer 12 x 8 mm großen asymmetrischen Membran (BTS 25, Memtec Timonium, Maryland, USA) aufgegeben. Die Remission auf der feinporigen Seite wurde nach acht Sekunden bei 567 nm gemessen. Bei 50 Messungen wurde ein Mittelwert der Remission von 40,25 % mit einem Variationskoeffizient von 1,14 % beobachtet.

### Beispiel 5

Eine asymmetrisch poröse Membran (BTS 25, Memtec America Corp. Timonium, Maryland, USA) mit einer Breite von 28 cm wurde in einer Lösung von 1 Gew.-% Natriumdodecylsulfat, 0,1 N Phosphatpuffer pH 7 und 0,7 mmol/l Kaliumhexacyanoferrat (III) getränkt. Die Flüssigkeitsaufnahme betrug etwa 115 ml/m². Die flüssigkeitsbelastete Membran wurde 30 Minuten bei 50 °C getrocknet. Die trockene Membran wurde in 8 mm breite Streifen geschnitten, mit Hilfe eines doppelseitigen Klebebandes auf eine 420 µm dicke PVC-Folie, die im Bereich der Membranapplikation mit einem 6 mm-Durchmesser großen Loch versehen war, so aufgeklebt, daß im Bereich des Loches die Membran nicht von dem doppelseitigen Klebestreifen bedeckt wurde. Die Orientierung der asymmetrischen Membran wurde so gewählt, daß die feinporige Seite zum Loch zeigte (vgl. Fig. 4). Anschließend wurden im Bereich des Loches ca. 10 µl Vollblut auf die großporige Seite der Membran aufgegeben. Der getüpfelte Teststreifen wurde in ein geeignetes Meßgerät eingeführt und nach 44 Sekunden auf der feinporigen Seite ausgemessen. Zwischen der Probenaufgabe und dem Meßzeitpunkt bildete sich auf der feinporigen Seite eine gleichmäßig dunkle Färbung aus. Obwohl der Testaufbau keinerlei zusätzliche Maßnahmen zur Verteilung des Blutes auf der Aufgabenseite beinhaltete, ergab sich eine unerwartet hohe Präzision der Ergebnisse. Der Variationskoeffizient betrug 1,6 % bei n = 10 (n = Anzahl der Messungen).

### Beispiel 6

Ein Teststreifenaufbau analog Beispiel 5 wurde realisiert mit dem Unterschied, daß die großporige Seite der Membran zum Loch zeigte (vgl. Fig. 5). Die Aufgabe des Blutes erfolgte durch das Loch in der Trägerfolie. Dies erleichterte etwas die Treffgenauigkeit bei der Probenaufgabe. Gemessen wurde von der feinporigen Seite her. Die Ergebnisse waren vergleichbar mit denen aus Beispiel 5.

### Beispiel 7

Ein Teststreifenaufbau analog Beispiel 5 (entspricht Fig. 4) wurde realisiert mit dem Unterschied, daß die Tränklösung die folgende Zusammensetzung hatte:
0,3 Gew.-% Natriumdioctylsulfosuccinat (DONS) (E. Merck, Darmstadt, Deutschland)
0,3 Gew.-% Natriumdiamylsulfosuccinat (DANS) (Cyanamid, Wolfratshausen/Obb., Deutschland)
0,2 Gew.-% Saponin
0,2 Gew.-% Kaliumhexacyanoferrat (III) in
0,1 mol/l Citratpuffer, pH 6,8

Die Durchführung der Messung entsprach Beispiel 5. Der Variationskoeffizient betrug in diesem Fall 1,25 % bei n = 10.

### Beispiel 8

Eine asymmetrisch poröse Membran (BTS 25) wurde in Teststreifen zwischen eine klare, transparente, 200 µm dicke Folie (Pokalon, Lonza, Weil am Rhein, Deutschland) und ein Glasfaservlies (Trapo 83/14, Fa. J.C. Binzer, Hatzfeld/Eder, Deutschland) so montiert, daß die großporige Seite zum Glasfaservlies zeigte, zwischen der Membran und dem Glasfaservlies aber keine Berührung stattfand (vgl. Fig. 7). Das Glasfaservlies wurde mit Hämiglobincyanid getränkt. Nach erfolgter Tränkung wurden die Teststreifen in einem geeigneten Gerät reflexionsphotometrisch bei 567 nm vermessen. Dabei wurde vor Meßbeginn durch die Meßeinrichtung der mechanische Kontakt zwischen dem getränkten Glasfaservlies und der asymmetrischen Membran hergestellt. Bei einer Hämiglobincyanidkonzentration von 7 g/dl wurde eine Remission von 32,69 % beobachtet. Der Variationskoeffizient betrug 1,77 %.

### Beispiel 9

Der Versuch nach Beispiel 8 wurde wiederholt, aber ohne die Membran zwischen dem Glasfaservlies und der transparenten Folie. Die Ergebnisse zeigten bei diesem Vorgehen einen Variationskoeffizienten von 5,84 %.

Ein Vergleich der Beispiele 8 und 9 zeigt deutlich, daß die verwendete Membran zu einer Vergleichmäßigung der Farbabbildung führt. Die Anwendung ist nicht auf analytische Verfahren zur Bestimmung des Hämoglobins im Blut beschränkt, sondern kann immer dann erfolgen, wenn es notwendig oder wünschenswert ist, die Homogenität einer Farbabbildung zu steigern.

### Beispiel 10

In Analogie zu Beispiel 3 wurde Bromthymolblau als sich nicht auf der feinporigen Seite einer asymmetrisch porösen Membran anreichernde Substanz untersucht. Nach Kontakt mit einer entsprechenden Farblösung wurde die getränkte und getrocknete BTS 25-Membran (Fa. Memtec Timonium, MD, USA) in flüssiges Paraffin (Schmelzpunkt 56 - 58 °C, Fa. Merck, Darmstadt, Deutschland) getaucht und anschließend abtropfen gelassen. Dadurch überzog sich die Membran mit einer dünnen Paraffinschicht, welche die Struktur stabilisiert, aber den Farbstoff oder die Membran selbst nicht anlöst.

Analog Beispiel 3 wurde ein Konzentrationsprofil erstellt. Figur 8 zeigt, daß die Farbintensität und damit die Farbkonzentration weitgehend konstant über die Membrandicke ist.

## Patentansprüche

1. Verwendung einer asymmetrisch porösen Membran zur gleichmäßig verteilten Anreicherung einer Substanz, die nicht oder nicht wesentlich an der Membran adsorbiert wird, in einer Membran-benetzenden Lösung auf der feinporigen Seite der Membran, wobei unter Membran-benetzend verstanden wird, daß die Zeit, die zum vollständigen Einsaugen eines Tropfens der Lösung mit einem Volumen von 20 µl, der auf ein 15×15 mm² großes Membranstück aufgegeben wird, benötigt wird, sowohl bei Aufgabe auf die grobporige als auch bei Aufgabe auf die feinporige Seite der Membran weniger als 20 s beträgt, und wobei unter nicht oder nicht wesentlich an der Membran adsorbiert verstanden wird, daß nach dem Durchsaugen von 8 ml einer Lösung der Substanz durch einen Stapel aus fünf Membranscheiben der asymmetrisch porösen Membran, wobei die Membranscheiben einen Durchmesser von 60 mm besitzen und die Lösung der Substanz auf die grobporige Seite der asymmetrisch porösen Membran aufgebracht wird, die Konzentration der Substanz im Filtrat verglichen mit der Konzentration der Substanz in der Ausgangslösung um weniger als 15 % abnimmt.

2. Verwendung gemäß Anspruch 1, dadurch gekennzeichnet, daß der Asymmetriefaktor der asymmetrisch porösen Membran größer als 10 ist.

3. Verfahren zur trägergebundenen Bestimmung einer Substanz in einer flüssigen Probe, bei dem eine Lösung der zu bestimmenden Substanz mit einer asymmetrisch porösen Membran kontaktiert oder die zu bestimmende Substanz in der Probe durch eine oder mehrere auf der Membran oder auf einer der Membran vorgelagerten Schicht vorliegende Stoffe freigesetzt oder gebildet wird und auf der Membran von der feinporigen Seite her bestimmt wird, wobei die zu bestimmende Substanz in der Membran-benetzenden Lösung nicht oder nicht wesentlich an der Membran adsorbiert wird und auf der feinporigen Seite der Membran gleichmäßig angereichert wird, wobei unter Membran-benetzend verstanden wird, daß die Zeit, die zum vollständigen Einsaugen eines Tropfens der Lösung mit einem Volumen von 20 µl, der auf ein 15×15 mm² großes Membranstück aufgegeben wird, benötigt wird, sowohl bei Aufgabe auf die grobporige als auch bei Aufgabe auf die feinporige Seite der Membran weniger als 20 s beträgt, und wobei unter nicht oder nicht wesentlich an der Membran adsorbiert verstanden wird, daß nach dem Durchsaugen von 8 ml einer Lösung der Substanz durch einen Stapel aus fünf Membranscheiben der asymmetrisch porösen Membran, wobei die Membranscheiben einen Durchmesser von 60 mm besitzen und die Lösung der Substanz auf die grobporige Seite der asymmetrisch porösen Membran aufgebracht wird, die Konzentration der Substanz im Filtrat verglichen mit der Konzentration der Substanz in der Ausgangslösung um weniger als 15 % abnimmt.

4. Verfahren gemäß Anspruch 3, dadurch gekennzeichnet, daß die asymmetrisch poröse Membran einen Asymmetriefaktor größer als 10 besitzt.

5. Verfahren gemäß Anspruch 3 oder 4, dadurch gekennzeichnet, daß die Porengröße auf der feinporigen Seite der asymmetrisch porösen Membran etwa 0.003 bis 3 µm beträgt.

6. Verfahren gemäß einem der Ansprüche 3 bis 5, dadurch gekennzeichnet, daß die asymmetrisch poröse Membran nach Benetzung durch das Lösungsmittel der zu untersuchenden flüssigen Probe noch mehr als 20% einer Meßstrahlung reflektiert.

7. Verfahren gemäß einem der Ansprüche 3 bis 6, dadurch gekennzeichnet, daß die zu bestimmende Substanz Hämoglobin oder ein Hämoglobinderivat ist.

8. Testträger zur Bestimmung einer Substanz in einer flüssigen Probe, wobei der Testträger eine asymmetrisch poröse Membran enthält, dadurch gekennzeichnet, daß die Substanz Hämoglobin ist, welches in der Membran-benetzenden wässrigen Lösung nicht oder nicht wesentlich an die Membran adsorbiert wird, auf der feinporigen Seite der Membran gleichmäßig verteilt angereichert wird und von der Feinporigen Seite her bestimmt werden kann, wobei unter Membran-benetzend verstanden wird, daß die Zeit, die zum vollständigen Einsaugen eines Tropfens der Lösung mit einem Volumen von 20 µl, der auf ein 15×15 mm² großes Membranstück aufgegeben wird, benötigt wird, sowohl bei Aufgabe auf die grobporige als auch bei Aufgabe auf die feinporige Seite der Membran weniger als 20 s beträgt, und wobei unter nicht oder nicht wesentlich an der Membran adsorbiert verstanden wird, daß nach dem Durchsaugen von 8 ml einer Lösung der Substanz durch einen Stapel aus fünf Membranscheiben der asymmetrisch porösen Membran, wobei die Membranscheiben einen Durchmesser von 60 mm besitzen und die Lösung der Substanz auf die grobporige Seite der asymmetrisch porösen Membran aufgebracht wird, die Konzentration der Substanz im Filtrat verglichen mit der Konzentration der Substanz in der Ausgangslösung um weniger als 15 % abnimmt, und wobei die Membran selbst eine hämolysierende Substanz trägt oder eine solche Substanz in einer vorgelagerten Schicht enthält.

9. Testträger gemäß Anspruch 8, dadurch gekennzeichnet, daß die asymmetrisch poröse Membran zusätzlich eine Substanz trägt, die zur Oxidation oder Komplexierung freien Hämoglobins führt.

10. Testträger gemäß einem der Ansprüche 8 bis 9 dadurch gekennzeichnet, daß die asymmetrisch poröse Membran auf einem festen Material so befestigt ist, daß die Aufgabe der zu bestimmenden Substanz in der Probe oder die Bestimmung der Substanz in der Probe auf der Seite der asymmetrisch porösen Membran erfolgt, die dem festen Material zugewandt ist.

11. Testträger gemäß Anspruch 10, dadurch gekennzeichnet, daß das feste Material durchscheinend ist.

12. Testträger gemäß Anspruch 10, dadurch gekennzeichnet, daß das feste Material ein Loch enthält, über dem die asymmetrisch poröse Membran befestigt ist.

## Claims

1. Use of an asymmetric porous membrane for the evenly distributed concentration of a substance which, in a membrane-wetting solution, is not or not substantially adsorbed to the membrane on the fine-pored side of the membrane wherein membrane-wetting is understood to mean that the time required to completely imbibe a drop of the solution with a volume of 20 µl applied to a 15x15 mm² piece of membrane is less than 20 s when applied to the coarsely-pored or to the fine-pored side of the membrane, and wherein not or not substantially adsorbed to the membrane is understood to mean that after 8 ml of a solution of the substance has been sucked through a pile of five membrane disks of the asymmetric porous membrane, the membrane disks having a diameter of 60 mm and the solution of the substance being applied to the coarse-pored side of the asymmetic porous membrane, the concentration of the substance in the filtrate compared with the concentration of the substance in the initial solution decreases by less than 15 %.

2. Use as claimed in claim 1, wherein the asymmetry factor of the asymmetric porous membrane is larger than 10.

3. Method for the carrier-bound determination of a substance in a liquid sample in which a solution of the substance to be determined is contacted with an asymmetric porous membrane or the substance to be determined in the sample is released or formed by one or several substances present on the membrane or on a layer located in front of the membrane and is determined on the membrane from the fine-pored side, wherein the substance to be determined in the membrane-wetting solution is not or not substantially adsorbed to the membrane and is evenly concentrated on the fine-pored side of the membrane whereby membrane-wetting is understood to mean that the time required to completely imbibe a drop of the solution with a volume of 20 µl applied to a 15x15 mm² piece of membrane is less than 20 s when applied to the coarse-pored or to the fine-pored side of the membrane, and whereby not or not substantially adsorbed to the membrane is understood to mean that after 8 ml of a solution of the substance has been sucked through a pile of five membrane disks of the asymmetric porous membrane, the membrane disks having a diameter of 60 mm and the solution of the substance being applied to the coarse-pored side of the asymmetic porous membrane, the concentration of the substance in the filtrate compared with the concentration of the substance in the initial solution decreases by less than 15 %.

4. Method as claimed in claim 3, wherein the asymmetric porous membrane has an asymmetry factor larger than 10.

5. Method as claimed in claim 3 or 4, wherein the pore size on the fine-pored side of the asymmetric porous membrane is about 0.003 to 3 µm.

6. Method as claimed in one of the claims 3 to 5, wherein the asymmetric porous membrane still reflects more than 20 % of a measurement radiation after being wetted with the solvent of the liquid sample to be examined.

7. Method as claimed in one of the claims 3 to 6, wherein the substance to be determined is haemoglobin or a haemoglobin derivative.

8. Test carrier containing an asymmetric porous membrane for the determination of a substance in a liquid sample, wherein the substance is haemoglobin which, in the membrane-wetting aqueous solution, is not or not substantially adsorbed to the membrane, is concentrated in an even distribution on the fine-pored side of the membrane and can be determined from the fine-pored side whereby membrane-wetting is understood to mean that the time required to completely imbibe a drop of the solution with a volume of 20 µl which is applied to a 15x15 mm² piece of membrane is less than 20 s when applied to the coarse-pored or to the fine-pored side of the membrane, and whereby not or not substantially adsorbed to the membrane is understood to mean that after 8 ml of a solution of the substance has been sucked through a pile of five membrane disks of the asymmetric porous membrane, the membrane disks having a diameter of 60 mm and the solution of the substance being applied to the coarse-pored side of the asymmetic porous membrane, the concentration of the substance in the filtrate compared with the concentration of the substance in the initial solution decreases by less than 15 % and wherein the membrane itself carries a haemolysing substance or contains such a substance in a layer located in front of it.

9. Test carrier as claimed in claim 8, wherein the asymmetric porous membrane in addition carries a substance which leads to the oxidation or complexing of free haemoglobin.

10. Test carrier as claimed in one of the claims 8 to 9, wherein the asymmetric porous membrane is mounted on a solid material in such a way that the application of the substance to be determined in the sample or the determination of the substance in the sample is carried out on the side of the asymmetric porous membrane which faces the solid material.

11. Test carrier as claimed in claim 10, wherein the solid material is translucent.

12. Test carrier as claimed in claim 10, wherein the solid material has a hole above which the asymmetric porous membrane is mounted.

## Revendications

1. Utilisation d'une membrane à porosité asymétrique pour l'enrichissement réparti de manière uniforme d'une substance qui ne s'est pas ou qui ne s'est essentiellement pas adsorbée sur la membrane, dans une solution mouillant la membrane sur le côté microporeux de la membrane, dans laquelle, par l'expression "mouillant la membrane", on veut dire que le temps requis pour l'absorption complète d'une goutte de la solution avec un volume de 20 µl, qui est alimentée sur un morceau de membrane possédant une dimension de 15 x 15 mm², représente moins de 20 secondes aussi bien lors d'une alimentation sur le côté macroporeux de la membrane que lors d'une alimentation sur le côté microporeux de la membrane, et dans laquelle, par l'expression "qui ne s'est pas ou qui ne s'est essentiellement pas adsorbée sur la membrane", on veut dire qu'après la pénétration par absorption de 8 ml d'une solution de la substance à travers une pile de cinq disques de la membrane à porosité asymétrique, les disques de la membrane possédant un diamètre de 60 mm et la solution de la substance étant appliquée sur le côté macroporeux de la membrane à porosité asymétrique, la concentration de la substance dans le filtrat, comparée à la concentration de la substance dans la solution de départ, diminue de moins de 15%.

2. Utilisation selon la revendication 1, caractérisée en ce que le facteur d'asymétrie de la membrane à porosité asymétrique est supérieur à 10.

3. Procédé pour l'analyse liée à un support d'une substance dans un échantillon liquide, dans lequel une solution de la substance à analyser entre en contact avec une membrane à porosité asymétrique ou bien la substance à analyser dans l'échantillon est libérée ou est formée via une ou plusieurs substances présentes sur la membrane ou sur une couche montée avant la membrane et est analysée sur la membrane à partir du côté microporeux, dans lequel la substance à analyser dans la solution mouillant la membrane ne s'est pas ou ne s'est essentiellement pas adsorbée sur la membrane et est enrichie de manière uniforme sur le côté microporeux de la membrane, dans lequel, par l'expression "mouillant la membrane", on veut dire que le temps requis pour l'absorption complète d'une goutte de la solution avec un volume de 20 µl, qui est alimentée sur un morceau de membrane possédant une dimension de 15 x 15 mm², représente moins de 20 secondes aussi bien lors d'une alimentation sur le côté macroporeux de la membrane que lors d'une alimentation sur le côté microporeux de la membrane, et dans lequel, par l'expression "qui ne s'est pas ou qui ne s'est essentiellement pas adsorbée sur la membrane", on veut dire qu'après la pénétration par absorption de 8 ml d'une solution de la substance à travers une pile de cinq disques de la membrane à porosité asymétrique, les disques de la membrane possédant un diamètre de 60 mm et la solution de la substance étant appliquée sur le côté macroporeux de la membrane à porosité asymétrique, la concentration de la substance dans le filtrat, comparée à la concentration de la substance dans la solution de départ, diminue de moins de 15%.

4. Procédé selon la revendication 3, caractérisé en ce que la membrane à porosité asymétrique possède un facteur d'asymétrie supérieur à 10.

5. Procédé selon la revendication 3 ou 4, caractérisé en ce que la grosseur des pores du côté microporeux de la membrane à porosité asymétrique s'élève d'environ 0,003 à 3 µm.

6. Procédé selon l'une quelconque des revendications 3 à 5, caractérisé en ce que la membrane à porosité asymétrique, après le mouillage par le solvant de l'échantillon liquide à analyser, réfléchit encore plus de 20% d'un faisceau de mesure.

7. Procédé selon l'une quelconque des revendications 3 à 6, caractérisé en ce que la substance à analyser est l'hémoglobine ou un dérivé de l'hémoglobine.

8. Support d'essai pour l'analyse d'une substance dans un échantillon liquide, le support d'essai contenant une membrane à porosité asymétrique, caractérisé en ce que la substance est l'hémoglobine, qui ne s'est pas ou ne s'est essentiellement pas adsorbée sur la membrane dans la solution aqueuse mouillant la membrane, qui est enrichie de manière uniforme sur le côté microporeux de la membrane et qui peut être analysée à partir du côté microporeux, dans lequel, par l'expression "mouillant la membrane", on veut dire que le temps requis pour l'absorption complète d'une goutte de la solution avec un volume de 20 µl, qui est alimentée sur un morceau de membrane possédant une dimension de 15 x 15 mm², représente moins de 20 secondes aussi bien lors d'une alimentation sur le côté macroporeux de la membrane que lors d'une alimentation sur le côté microporeux de la membrane, et dans lequel, par l'expression "qui ne s'est pas ou qui ne s'est essentiellement pas adsorbée sur la membrane", on veut dire qu'après la pénétration par absorption de 8 ml d'une solution de la substance à travers une pile de cinq disques de la membrane à porosité asymétrique, les disques de la membrane possédant un diamètre de 60 mm et la solution de la substance étant appliquée sur le côté macroporeux de la membrane à porosité asymétrique, la concentration de la substance dans le filtrat, comparée à la concentration de la substance dans la solution de départ, diminue de moins de 15%, et dans lequel la membrane elle-même porte une substance ayant un effet d'hémolyse ou contient une substance de ce type dans une couche montée en amont.

9. Support d'essai selon la revendication 8, caractérisé en ce que la membrane à porosité asymétrique porte en outre une substance qui donne lieu à l'oxydation ou à la complexation de l'hémoglobine libre.

10. Support d'essai selon l'une quelconque des revendications 8 à 9, caractérisé en ce que la membrane à porosité asymétrique est fixée sur une matière solide de telle sorte que l'alimentation de la substance à analyser dans l'échantillon ou l'analyse de la substance dans l'échantillon a lieu sur le côté de la membrane à porosité asymétrique qui est orienté vers la matière solide.

11. Support d'essai selon la revendication 10, caractérisé en ce que la matière solide est transparente.

12. Support d'essai selon la revendication 10, caractérisé en ce que la matière solide contient un trou à travers lequel est fixée la membrane à porosité asymétrique.
